# EUROPEAN PATENT SPECIFICATION

(11) **EP 0 633 892 B1**
(45) Date of publication and mention of the grant of the patent: **26.05.1999**
(21) Application number: 93911567.1
(22) Date of filing: 24.03.1993
(51) Int. Cl.: C07J 41/00, C07J 75/00

(54) **N-T-BUTYL-ANDROST-3,5-DIENE-17-beta-CARBOXAMIDE-3-CARBOXYLIC ACID POLYMORPH A**
N-T-BUTYL-ANDROST-3, 5-DIEN-17-beta-CARBOXAMID-3-CARBONSÄURE POLYMORPHA
POLYMORPHE A D'ACIDE N-T-BUTYL-ANDROST-3,5-DIENE-17-beta-CARBOXAMIDE-3-CARBOXYLIQUE

(30) Priority: 24.03.1992 GB 9206413
(43) Date of publication of application: 18.01.1995
(73) Proprietor: SMITHKLINE BEECHAM CORPORATION, Philadelphia Pennsylvania 19101 (US)
(72) Inventor: BAINE, Neil, Howard, Merion, PA 19066 (US); HOLDER, Neville, Lewis, Cherry Hill, NJ 08034 (US); KLINE, Donald, Nathaniel, Audubon, PA 19403 (US); WEBB, Robert, Lee, West Chester, PA 19380 (US); ZUBER, Gary, Edward, Audubon, PA 19403 (US)
(74) Representative: Giddings, Peter John, Dr.
(86) International application number: US9302974
(87) International publication number: WO9319081

(56) References cited:
- EP-A- 0 289 327
- Endocrinology, 1992, LAMB et al., "Prostatic Involution in Rats Induced by a Novel 5 - Reductase Inhibitor, SK&F 105657: Role for Testosterone in the Androgenic Response", pp. 685-694, see 686.
- Proceedings of the National Academy of Sciences, 1992, HARRIS et al., "Identification and Selective Inhibition of an Isozyme of Steroid 5 - Reductase in Human Scalp", pp. 10787-10791, see p. 10789.
- Bioorganic Chemistry, 1989, METCALF et al., "Potent Inhibition of Human Steroid 5 - Reductase by 3-Androstene -3- Carboxylic Acids", pp. 372-376, see p. 374.
- Journal of Steroid Biochemistry, 1989, LEVY et al., "Interaction Between Rat Prostatic Steroid 5 - Reductase and 3-Carboxy-17B-Substituted Steroids: Novel Mechanism of Enzyme Inhibition", pp. 571-575, see 572-3.
- Biochemistry, 1990, LEVY et al., "Inhibition of Rat Liver Steroid 5 - Reductase by 3-Androstene-3- Carboxylic Acids: Mechanism of Enzyme Inhibitor Interaction", pp. 2815-2823, see p. 2817.
- Bioorganic Chemistry, 1991, LEVY et al., "3-Phosphinic Acid and 3-Phosphonic Acid Steroids as Inhibitors of Steroid 5 - Reductase: Species Comparison and Mechanistic Studies", pp. 245-260, see pp. 246, 254.
- Journal of Medicinal Chemistry, 1990, HOLT et al., "Inhibition of Steroid 5 - Reductase by Unsaturated 3-Carboxy Steroids", pp. 943-950, see p. 945.

## Description

The present invention relates to a novel polymorphic form of N-t-butyl-androst-3,5-diene-17β-carboxamide-3-carboxylic acid.

### Brief Description of the Drawings

Figure I is an Infra-red Spectrum of N-t-butyl-androst-3,5-diene-17β-carboxylic acid polymorph A.

Figure II is an enhanced FT-IR spectra of the 3399-3501 cm⁻¹ region of Polymorph A disclosing the characteristic N-H stretch of Polymorph A.

### Detailed Description of the Invention

N-t-butyl-androst-3,5-diene-17β-carboxamide-3-carboxylic acid is a compound which is disclosed and claimed as being useful in the treatment of benign prostatic hypertrophy in U.S. Patent No. 5,017,568, (European 0289327A). Said compound can be prepared by methods such as described in U.S. Patent No. 5,017,568: Example 28(iv) describes the product being recrystallised by dissolving a reaction product in ethyl ether, adding ethyl acetate and concentration. Example 28 B (iv) describes the product being recrystallised from ethyl acetate at ambient temperatures. The isolation and identification of the polymorphic forms of said compound is advantageous in identifying desirable physical characteristics of the different crystal forms of said compound.

It has now been found that a polymorphic form (hereinafter Polymorph A) of the compound N-t-butyl-androst-3,5-diene-17β-carboxamide-3-carboxylic acid can be obtained in a high state of polymorphic purity by stirring a 12-40% by weight slurry of N-t-butyl-androst-3,5-diene-17β-carboxamide-3-carboxylic acid in ethyl acetate for about an hour, at a temperature above 60°C, then stirring said slurry at a temperature of from about 0-5°C with subsequent isolation of the Polymorph A.

Presently, N-t-butyl-androst-3,5-diene-17β-carboxamide-3-carboxylic acid is known to assume only two polymorphic forms, A and B.

Polymorph A and Polymorph B were individually subjected to intense grinding in a mortar with a pestle for a period of approximately five minutes. Infra-red spectral absorbencies of the post-grinding Polymorph B compound indicated that approximately 5-10% of said polymorph had converted to the Polymorph A form. Infra-red spectral absorbencies of the post-grinding Polymorph A compound indicated retentiqn of polymorphic identity and purity. The above findings indicate that the polymorphic A form of N-t-butyl-androst-3,5-diene-17β-carboxamide-3-carboxylic acid is thermodynamically more stable than the polymorphic B form. The themodynamically more stable polymorphic form of a compound is advantageous for maintaining crystal integrity during manufacture, storing, shipping and handling of solid compositions of said compound. Since, as described above, the polymorphic B form of N-t-butyl-androst-3,5-diene-17β-carboxamide-3-carboxylic acid does not retain its crystal integrity upon grinding and the polymorphic A form retains crystal integrity, Polymorph A is particularly advantageous in the manufacture of tableted forms of N-t-butyl-androst-3,5-diene-17β-carboxamide-3-carboxylic acid.

By the term "crude" as used herein is meant that the isolated N-t-butyl-androst-3,5-diene-17β-carboxamide-3-carboxylic acid starting material exist as an amorphous solid, in an undesired polymorphic form or as a plurality of polymorphic forms.

By the term "N-t-butyl-androst-3,5-diene-17β-carboxamide-3-carboxylic acid" as used herein is meant a compound of the structure

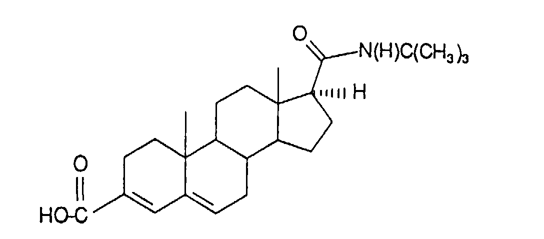

N-t-butyl-androst-3,5-diene-17β-carboxamide-3-carobxylic acid polymorph A (prepared by crystallization from ethyl acetate) was analyzed by an X-ray powder diffraction (X-ray diffractometry (XRD) obtained from Micron Incorporated of Wilmington, Delaware). The characteristic d-spacings, intensities, and 2-theta values for the diffraction pattern of Polymorph A are listed in Table 1 below.

**Table 1**

| X-Ray diffraction pattern listing of N-t-butyl-androst-3,5-diene-17β-carboxamide-3-carboxylic acid Polymorph A. | | | | |
|---|---|---|---|---|
| | D | I/IMAX | 2Theta | PK WIDTH |
| 1 | 19.12558 | 76.46 | 4.620 | 0.00 |
| 2 | 10.64122 | 2.44 | 8.309 | 0.00 |
| 3 | 9.42222 | 6.60 | 9.386 | 0.00 |
| 4 | 8.72598 | 2.26 | 10.137 | 0.00 |
| 5 | 7.47791 | 12.67 | 11.835 | 0.00 |
| 6 | 6.80494 | 19.00 | 13.010 | 0.00 |
| 7 | 6.14523 | 27.60 | 14.413 | 0.00 |
| 8 | 5.95738 | 47.09 | 14.871 | 0.00 |
| 9 | 5.49032 | 35.12 | 16.144 | 0.00 |
| 10 | 4.91804 | 34.76 | 18.037 | 0.00 |
| 11 | 4.44050 | 25.31 | 19.996 | 0.00 |
| 12 | 4.28147 | 14.11 | 20.746 | 0.00 |
| 13 | 4.09672 | 14.41 | 21.693 | 0.00 |
| 14 | 4.01321 | 19.43 | 22.150 | 0.00 |
| 15 | 3.71622 | 6.71 | 23.946 | 0.00 |
| 16 | 3.47394 | 6.11 | 25.643 | 0.00 |
| 17 | 3.30066 | 4.51 | 27.014 | 0.00 |
| 18 | 3.23171 | 4.04 | 27.602 | 0.00 |
| 19 | 2.75384 | 1.93 | 32.514 | 0.00 |
| 20 | 2.67300 | 2.36 | 33.526 | 0.00 |
| 21 | 2.51415 | 1.96 | 35.713 | 0.00 |
| 22 | 2.20969 | 2.68 | 40.838 | 0.00 |

The following example illustrates preparation of N-t-butyl-androst-3,5-diene-17β-carboxamide-3-carboxylic acid polymorph A. The example is not intended to limit the scope of the invention as defined hereinabove and as claimed below.

### Example 1

### N-t-butyl-androst-3,5-diene-17β-carboxamide-3-carboxylic acid Polymorph A

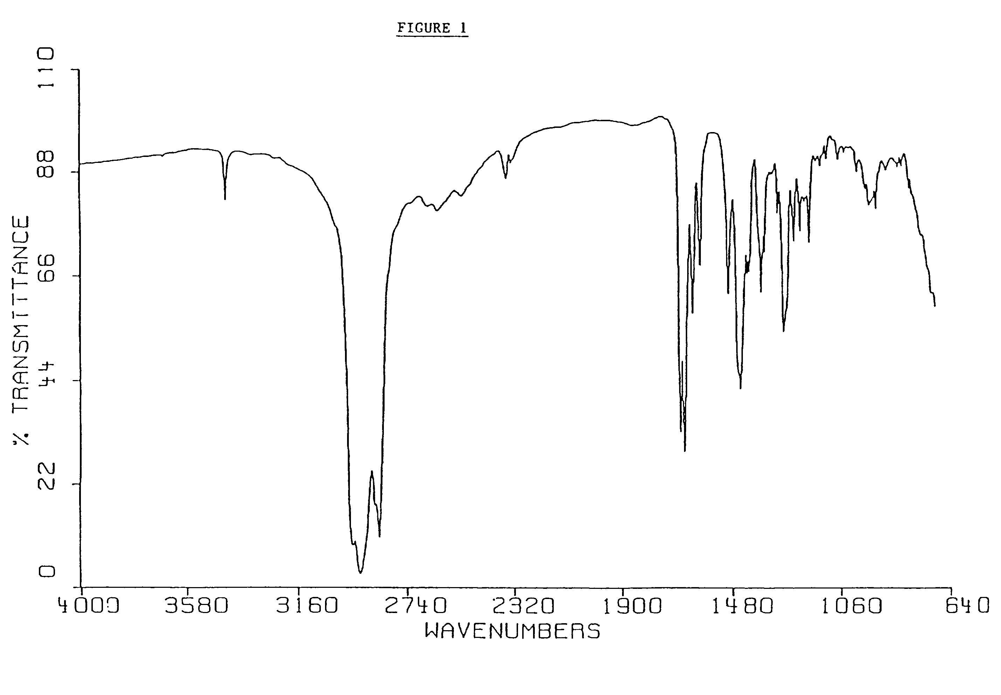

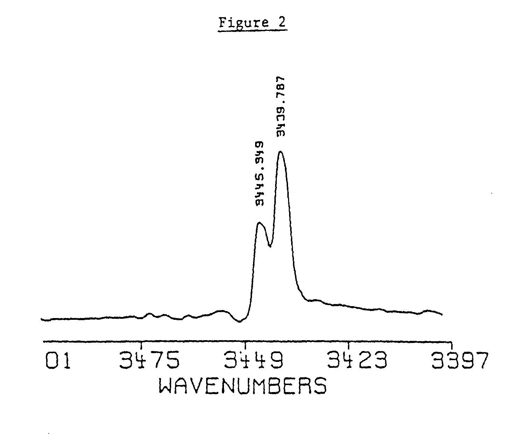

6.4 g of crude N-t-butyl-androst-3,5-diene-17β-carboxamide-3-carboxylic acid was added to 38.4 ml of ethyl acetate. The resulting slurry was warmed, with stirring, at about 65-70°C for about two hours. The resulting slurry was cooled to about 0-5°C for 1 hour, and was filtered and washed with 5-10 ml of cold ethyl acetate. The product was filtered dried under vacuum at about 50°C to afford 5 grams of substantially pure N-t-butyl-androst-3,5-diene-17β-carboxamide-3-carboxylic acid in its polymorph form. The infra-red spectrum of the product is shown in Figures 1 and 2.

Infra-red spectral absorbancies of N-t-butyl-androst-3,5-diene-17β-carboxamide-3-carboxylic acid polymorph A (prepared by crystallization from ethyl acetate) were obtained. (spectrum obtained from Nujol (mineral oil) on sodium chloride plates) (apparatus, Nicolet 6000 FT-IR using a mercury cadmium telluride detector, analysis time 7.6 minutes (800 scans), Enhancement Program Nicolet IRDCON).

Characteristic polymorph A form bands occur at 3441 cm⁻¹ (N-H stretch); 1678 cm⁻¹ (acid C=O stretch); and 1662 cm⁻¹ (amide C=O stretch). An FT-IR spectra of polymorph A is shown in Figure 1 below. The resolution enhanced FT-IR spectra of the 3399-3501 cm⁻¹ region disclosing said characteristic N-H stretch of polymorph A is shown in Figure 2 below.

## Claims

1. A compound of the structure 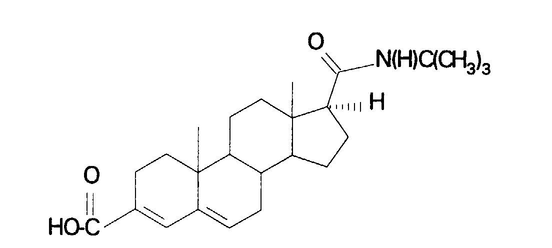 substantially in the polymorph A form and having the infra-red spectrum as shown in Figure 1.

2. A compound according to claim 1 having the infra-red spectrum as shown in Figure 2.

3. A compound according to claim 1 or claim 2 having the x-ray diffraction pattern listing shown in Table 1.

4. A process for preparing a compound of the structure 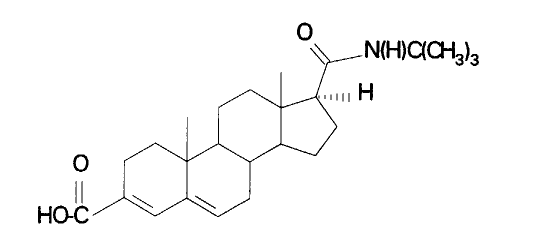 substantially in the polymorph A form, which comprises stirring a 12-40% by weight slurry of crude N-t-butyl-androst-3,5-diene-17β-carboxamide-3-carboxylic acid in ethyl acetate for about an hour at a temperature above 60°C, then stirring said slurry at a temperature from about 0-5°C with subsequent isolation of Polymorph A.

5. A process according to claim 4 which comprises stirring a 14-20% by weight slurry of the crude N-t-butyl-androst-3, 5-diene-17β-carboxamide-3-carboxylic acid in ethyl acetate for over an hour at a temperature above 60°C, then stirring said slurry at a temperature from about 0-5°C with subsequent isolation of Polymorph A.

## Patentansprüche

1. Verbindung der Struktur 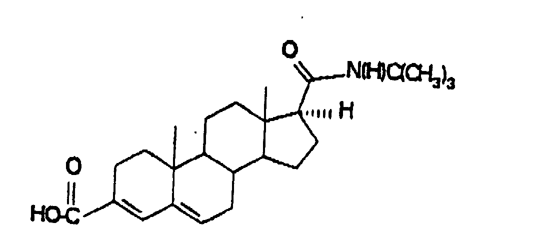 die im wesentlichen in Form des Polymorphs A vorliegt und ein Infrarotspektrum wie in Fig. 1 gezeigt aufweist.

2. Verbindung gemäß Anspruch 1, die ein Infrarotspektrum gemäß Fig. 2 aufweist.

3. Verbindung gemäß Anspruch 1 oder Anspruch 2, die das Röntgenbeugungsmuster aufweist, dessen Signale in Tabelle 1 aufgeführt sind.

4. Verfahren zur Herstellung einer Verbindung der Struktur 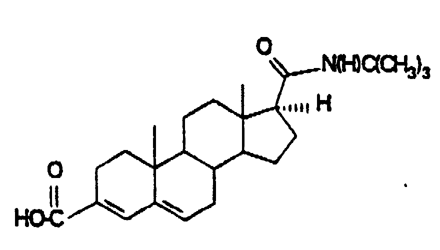 die im wesentlichen in Form des Polymorphs A vorliegt, umfassend das Rühren einer 12-40gew.-%igen Aufschlämmung roher N-t-Butyl-androst-3,5-dien-17β-carboxamid-3-carbonsäure in Ethylacetat ungefähr 1 Stunde lang bei einer Temperatur oberhalb von 60°C, danach Rühren der Aufschlämmung bei einer Temperatur von ungefähr 0 bis 5°C mit anschließender Isolation des Polymorphs A.

5. Verfahren gemäß Anspruch 4, umfassen das Rühren einer 14-20gew.-%igen Aufschlämmung der rohen N-t-Butyl-androst-3,5-dien-17β-carboxamid-3-carbonsäure in Ethylacetat ungefähr 1 Stunde lang bei einer Temperatur oberhalb von 60°C, danach Rühren der Aufschlämmung bei einer Temperatur von ungefähr 0 bis 5°C mit anschließender Isolation des Polymorphs A.

## Revendications

1. Composé de formule structurale 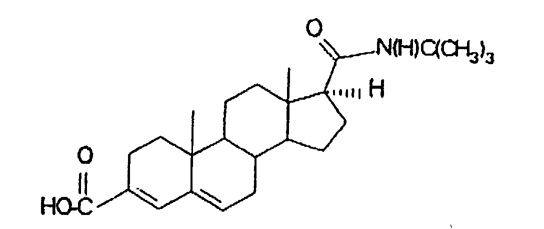 essentiellement sous la forme polymorphe A et ayant le spectre infrarouge représenté sur la figure 1.

2. Composé suivant la revendication 1, ayant le spectre infrarouge représenté sur la figure 2.

3. Composé suivant la revendication 1 ou la revendication 2, ayant les résultats de diagramme de diffraction des rayons X énumérés sur le tableau 1.

4. Procédé pour la préparation d'un composé de formule structurale 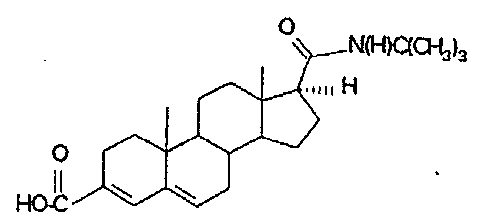 essentiellement sous la forme polymorphe A, qui comprend l'agitation d'une suspension à 12-40 % en poids d'acide N-tertio-butyl-androst-3,5-diène-17β-carboxamide-3-carboxylique brut dans de l'acétate d'éthyle pendant environ une heure à une température supérieure à 60°C, puis l'agitation de ladite suspension à une température d'environ 0 à 5°C avec isolement ultérieur de la forme polymorphe A.

5. Procédé suivant la revendication 4, qui comprend l'agitation d'une suspension à 14-20 % en poids de l'acide N-tertio-butyl-androst-3,5-diène-17β-carboxamide-3-carboxylique brut dans de l'acétate d'éthyle pendant un temps supérieur à une heure à une température supérieure à 60°C, puis l'agitation de ladite suspension à une température d'environ 0 à 5°C avec isolement ultérieur de la forme polymorphe A.
